(19) **Europäisches Patentamt**
**European Patent Office**
**Office européen des brevets**

(11) **EP 2 950 713 B1**

(12) # FASCICULE DE BREVET EUROPEEN

(45) Date de publication et mention
de la délivrance du brevet:
**26.08.2020 Bulletin 2020/35**

(21) Numéro de dépôt: **14706105.5**

(22) Date de dépôt: **29.01.2014**

(51) Int Cl.:
**A61B 5/0484** *(2006.01)* **G06F 3/01** *(2006.01)*

(86) Numéro de dépôt international:
**PCT/IB2014/058635**

(87) Numéro de publication internationale:
**WO 2014/118711 (07.08.2014 Gazette 2014/32)**

(54) **PROCEDE DE LOCALISATION D'UNE ACTIVITE CEREBRALE, NOTAMMENT POUR COMMANDE NEURONALE DIRECTE**

VERFAHREN ZUR ORTUNG EINER HIRNAKTIVITÄT, BESONDERS FÜR DIREKTE NEURALE STEUERUNG

METHOD FOR LOCATING A BRAIN ACTIVITY, IN PARTICULAR FOR DIRECT NEURAL CONTROL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.01.2013 FR 1350783**

(43) Date de publication de la demande:
**09.12.2015 Bulletin 2015/50**

(73) Titulaire: **Commissariat à l'Énergie Atomique et aux Énergies Alternatives 75015 Paris (FR)**

(72) Inventeurs:
• **AKSENOVA, Tetiana**
**38120 St. Egreve (FR)**
• **LABYT, Etienne**
**38950 St. Martin De Vinoux (FR)**
• **MISHCHENKO, Ales**
**St. Petersbourg 192283 (RU)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
**WO-A1-2012/068493 US-A- 4 736 751**
**US-A- 5 447 166 US-A1- 2002 042 563**
**US-A1- 2012 035 765**

• **KUANGDA LI ET AL: "Correlation Between Forehead EEG and Sensorimotor Area EEG in Motor Imagery Task", DEPENDABLE, AUTONOMIC AND SECURE COMPUTING, 2009. DASC '09. EIGHTH IEEE INTERNATIONAL CONFERENCE ON, IEEE, PISCATAWAY, NJ, USA, 12 décembre 2009 (2009-12-12), pages 430-435, XP031610025, ISBN: 978-0-7695-3929-4**
• **ARPAN BANERJEE ET AL: "Spatiotemporal re-organization of large-scale neural assemblies underlies bimanual coordination", NEUROIMAGE, ACADEMIC PRESS, ORLANDO, FL, US, vol. 62, no. 3, 20 mai 2012 (2012-05-20), pages 1582-1592, XP028449958, ISSN: 1053-8119, DOI: 10.1016/J.NEUROIMAGE.2012.05.046 [extrait le 2012-05-25]**
• **PIROSKA H ET AL: "Specific Movement Detection in EEG Signal Using Time-Frequency Analysis", COMPLEXITY AND INTELLIGENCE OF THE ARTIFICIAL AND NATURAL COMPLEX SYSTEMS, MEDICAL APPLICATIONS OF THE COMPLEX SYSTEMS, BIOMEDICAL COMPUTING, 2008. CANS '08. FIRST INTERNATIONAL CONFERENCE ON, IEEE, PISCATAWAY, NJ, USA, 8 novembre 2008 (2008-11-08), pages 209-215, XP031525246, ISBN: 978-0-7695-3621-7**

• Alexandre Barachant: "Filtrage spatial robuste à partir d'un sous-ensemble optimal d'électrodes en BCI EEG", Actes du Colloque GRETSI 2009, 8-11 septembre 2009, Dijon, France, 8 septembre 2009 (2009-09-08), XP055002259, Extrait de l'Internet: URL:http://documents.irevues.inist.fr/bits tream/handle/2042/28933/barachant_554.pdf? sequence=1 [extrait le 2011-07-07] cité dans la demande

• BASHASHATI A ET AL: "Comparison of Using Mono-Polar and Bipolar Electroencephalogram (EEG) Electrodes for Detection of Right and Left Hand Movements in a Self-Paced Brain Computer Interface (BCI)", ELECTRICAL AND COMPUTER ENGINEERING, 2007. CCECE 2007. CANADIAN CONFER ENCE ON, IEEE, PI, 26 avril 2007 (2007-04-26), pages 725-728, XP031176628, ISBN: 978-1-4244-1020-0

**Description**

L'invention porte sur un procédé de localisation de l'activité cérébrale d'un sujet, notamment par magnétoencéphalographie. L'invention s'applique en particulier au domaine de la commande neuronale directe.

La commande neuronale directe (BCI, de l'anglais « *brain-computer interface* », ou interface cerveau-ordinateur) permet d'établir une communication entre un utilisateur et une machine (typiquement un ordinateur) à travers des signaux neuronaux issus de l'activité cérébrale d'un sujet sans recourir à la voie musculaire, ce qui constitue un réel espoir pour les personnes souffrant de graves paralysies.

Les systèmes non intrusifs de commande neuronale directe utilisent, le plus souvent, l'électroencéphalographie (EEG) comme méthode d'acquisition de l'activité cérébrale. On place ainsi un certain nombre d'électrodes à la surface du crâne dans le but d'y mesurer une activité électrique reflétant l'activité cérébrale du sujet. D'autres techniques, plus performantes mais aussi plus intrusives, exploitent des signaux électrocorticographiques (ECoG), prélevés à la surface du cortex, voire des signaux prélevés par des électrodes profondes. La magnétoencéphalographie (MEG) est une technique non intrusive, dont l'utilisation en commande neuronale directe est conceptuellement intéressante, car les signaux magnétiques ne subissent pas - ou peu - de distorsion lorsqu'ils se propagent à travers le crâne. Son principal inconvénient, qui en pratique la limite à des applications expérimentales, est l'insuffisante miniaturisation des capteurs magnétoencéphalographiques.

Quelle que soit la méthode d'acquisition de l'activité cérébrale utilisée, le principe à la base de la commande neuronale directe consiste généralement à associer une ou plusieurs tâches mentales (action imaginées par le sujet) à une ou plusieurs actions réalisées par un effecteur. Par exemple, l'imagination du mouvement de la main droite peut être associée au déplacement vers la droite d'un curseur.

La prise en compte de l'information spatiale véhiculée par les signaux neuronaux est importante pour réaliser cette association. En effet, l'effectuation de tâches mentales différentes active différentes régions du cerveau, ou les mêmes régions mais d'une manière différente. Pour préserver au maximum cette information spatiale on utilise, dans la plupart des cas, un grand nombre de capteurs (jusqu'à une centaine). Cette approche présente plusieurs inconvénients : une gêne pour l'utilisateur, un temps de préparation long, un coût calculatoire élevé. En outre, certains types de traitements montrent des limitations quand le nombre de capteurs augmente (par exemple, on observe des effets de sur-apprentissage). Ainsi, des techniques ont été développées pour déterminer les emplacements optimaux, sur le crâne ou à la surface du cortex d'un sujet, où situer un nombre aussi limité que possible de capteurs. Par exemple, l'article de A. Barachant, T. Aksenova, et S. Bonnet, « Filtrage spatial robuste à partir d'un sous-ensemble optimal d'électrodes en BCI EEG » GRETSI 2009, 8 - 11 septembre 2009, décrit une méthode de sélection ascendante (c'est-à-dire dans laquelle on construit progressivement un ensemble optimal de capteurs), basée sur un critère de corrélation multiple de la log-variance des signaux EEG après filtrage fréquentiel.

La demande de brevet FR2992543 A1 décrit un procédé de localisation de l'activité cérébrale d'un sujet impliqué dans une tâche, exploitant en particulier la magnétoencéphalographie. Ce procédé se base sur le calcul d'un coefficient de détermination exprimant la corrélation entre les signaux issus d'un capteur (constitué en particulier d'un magnétomètre et d'une paire de gradiomètres) et un vecteur d'observation indicatif de la présence d'un stimulus sensoriel qui déclenche l'effectuation de la tâche par le sujet. Les capteurs présentant les coefficients de détermination les plus élevés représentent les régions du cerveau les plus actives, qui peuvent être exploitées de manière préférentielle pour la réalisation d'une commande neuronale directe.

Les Documents US5447166A et US2002/0042563A1 divulguent de diverse procédés de localisation d'une activité cérébrale.

Les présents inventeurs se sont rendus compte que ce procédé, comme toutes les techniques connues de l'art antérieur et visant à établir une corrélation entre l'activité cérébrale (notamment corticale) et une tâche effectuée en réponse à un stimulus sensoriel, présentent l'inconvénient de détecter certaines régions du cerveau qui s'avèrent en réalité non-spécifiques à la tâche considérée. Les signaux issus de ces régions sont donc des signaux parasites, dont la prise en compte nuit à l'efficacité de la commande neuronale. Une étude a permis de déterminer que ces régions non-spécifiques ne sont pas activées par la tâche étudiée, mais par la perception du stimulus sensoriel ; il s'agit donc principalement des zones visuelles ou auditives du cortex, selon que le stimulus soit un signal visuel ou un son.

L'invention vise à surmonter cet inconvénient de l'art antérieur en permettant une meilleure discrimination entre régions du cerveau spécifiques et non-spécifiques à la tâche considérée. La description détaillée fournira plutôt à l'homme du métier une feuille de route adaptée pour implémenter un mode de réalisation exemplaire. Il est entendu que différentes modifications peuvent être réalisées, dans la portée de la matière revendiquée dans la présente, telle qu'exposée dans les revendications annexées.

Conformément à un mode de réalisation exemplaire, ce but est réalisé en faisant effectuer au sujet sous étude (généralement un être humain, mais dans certains cas il peut s'agir d'un animal dressé) non une, mais (au moins) deux tâches successives différentes entre elles, en réponse à des stimuli sensoriels respectifs. La prise en compte conjointe des signaux neuronaux acquis lors de l'effectuation des différentes tâches permet de s'affranchir de l'influence des régions cérébrales non-spécifiques, activées par la perception du stimulus plus que par les tâches elles-mêmes. Les deux stimuli sensoriels seront de même nature - par exemple, tous deux visuels ou tous deux sonores. De préférence, les deux tâches effectuées correspondront à des mouvements (réels ou imaginés) d'un membre droit du corps du sujet et du membre gauche correspondant.

Ainsi, un mode de réalisation exemplaire est un procédé de localisation d'une activité cérébrale, comportant les étapes suivantes :

a) Adresser à un sujet une première série de stimuli sensoriels et acquérir, au moyen d'un ensemble de capteurs, des premières séries respectives de signaux représentatifs d'une activité cérébrale associée à une première tâche effectuée ou imaginée par ledit sujet en réponse aux stimuli sensoriels de ladite première série, chaque dit capteur étant sensible à l'activité d'une région respective du cerveau dudit sujet ;

b) Adresser audit sujet une deuxième série de stimuli sensoriels et acquérir, au moyen dudit ensemble de capteurs, des deuxièmes séries respectives de signaux représentatifs d'une activité cérébrale associée à une deuxième tâche, différente de ladite première tâche, effectuée ou imaginée par ledit sujet en réponse aux stimuli sensoriels de ladite deuxième série ; et

c) Pour chaque dit capteur, construire une variable multidimensionnelle représentative de la première et de la deuxième série correspondantes de signaux, et déterminer un coefficient de corrélation entre ladite variable multidimensionnelle et un vecteur d'observation représentatif desdits premier et deuxième stimuli sensoriels.

Selon différents modes de réalisation exemplaires :

- Ladite première tâche peut correspondre à un mouvement d'un membre droit du corps dudit sujet et ladite deuxième tâche peut correspondre à un mouvement d'un membre gauche, ou vice-versa. Plus particulièrement, ladite première tâche peut correspondre à un mouvement d'un membre droit du corps dudit sujet et ladite deuxième tâche à un mouvement symétrique du membre gauche correspondant, ou vice-versa.
- Ladite étape c) peut comprendre la concaténation desdites première et deuxième série de signaux, avec changement de signe de l'une d'entre elles.
- Lesdits stimuli sensoriels desdites première et deuxième séries peuvent être de même nature.
- Ladite étape c) peut comprendre la réalisation d'une analyse temps-fréquence desdites séries de signaux, moyennant quoi ladite variable multidimensionnelle peut être une matrice.
- Ladite étape c) peut comprendre une opération de normalisation et centrage desdites séries de signaux.
- Lesdits capteurs peuvent être des capteurs magnétoencéphalographiques, et en particulier chacun desdits capteurs peut comprendre une paire de gradiomètres agencés pour acquérir deux composantes spatiales distinctes d'un gradient d'un champ magnétique généré par le cerveau dudit sujet.
- Le procédé peut comprendre également une étape d) de visualisation, pendant laquelle on projette des valeurs indicatives des coefficients de corrélations déterminés pour chaque dit capteur sur un modèle tridimensionnel d'une surface corticale, et l'on réalise une interpolation desdites valeurs entre différents points d'un maillage de ladite surface.

Un autre mode de réalisation exemplaire. est un procédé de localisation de capteurs d'activité cérébrale pour commande neuronale directe comportant :

- une étape de localisation d'une activité cérébrale, mise en œuvre par un procédé tel que défini ci-dessus ; et
- une étape de détermination de localisations optimales desdits capteurs d'activité cérébrale en fonction des résultats de ladite étape de localisation d'une activité cérébrale.

D'autres caractéristiques, détails et avantages de l'invention ressortiront à la lecture de la description faite en référence aux dessins annexés donnés à titre d'exemple et qui représentent, respectivement :

- Les figures 1A et 1B, des mappes des coefficients de corrélation entre un stimulus visuel (OK) et des signaux magnétoencéphalographiques acquis sur un sujet qui, en réponse à ce stimulus, imagine d'effectuer un mouvement de l'indice gauche et de l'indice droit, respectivement ; et
- La figure 2, des mappes de coefficients de corrélation obtenues par un procédé conformément à un mode de réalisation de l'invention, considérant conjointement les signaux magnétoencéphalographiques acquis en correspondance des deux tâches considérées.

A titre d'exemple non limitatif, l'invention sera décrite en référence à un mode de réalisation particulier, dans lequel les signaux représentatifs d'une activité cérébrale sont acquis au moyen de capteurs magnétoencéphalographiques constitués par deux gradiomètres sensibles à des composantes orthogonales entre elles et parallèles à la surface du crâne du gradient d'un champ magnétique généré par le cortex cérébral du sujet. Dans cet exemple, le stimulus est de type visuel et les deux tâches effectuées par le sujet consistent à imaginer un mouvement de frappe de l'index gauche ou droit, respectivement.

Pour la première tâche effectuée (mouvement imaginaire de l'indexe gauche), pour chaque capteur et pour chaque stimulus visuel on acquiert un signal représentatif d'une activité cérébrale du sujet ; comme en général chaque capteur comprend plusieurs capteurs élémentaires (dans le cas présent, deux gradiomètres), le signal présente plusieurs composantes. Une analyse temps-fréquence permet de représenter ce signal sous forme vectorielle : $x(t_i+\tau)=[x^1{}_{f1}(t_i+\tau)...$ $x^1{}_{fM}(t_i+\tau) ... x^{Nc}{}_{f1}(t_i+\tau)... x^{Nc}{}_{fM}(t_i+\tau)]^T$ où f1 - fM sont des composantes spectrales du signal, l'exposant de valeur compris entre 1 et $N_c$ identifie les composantes du signal provenant des différents capteurs élémentaires (ici : $N_c$=2), $t_i$ est l'instant auquel le i-ème stimulus est administré et $\tau$ est le temps d'acquisition (temps écoulé depuis l'instant $t_i$). La dimension de la variable vectorielle **x** est donc $N_cM$.

Cette opération est répétée une pluralité (N>1) de fois, et les vecteurs **x** ainsi obtenus sont utilisés pour construire la variable matricielle **X** définie de la manière suivante :

$$\mathbf{X} = \begin{pmatrix} 1 & x^1_{f1}(t_1+\tau) & x^1_{f2}(t_1+\tau) & ... & x^2_{f1}(t_1+\tau) & x^2_{f2}(t_1+\tau) & ... \\ 1 & x^1_{f1}(t_2+\tau) & x^1_{f2}(t_2+\tau) & ... & x^2_{f1}(t_2+\tau) & x^2_{f2}(t_2+\tau) & ... \\ ... & ... & ... & ... & ... & ... & ... \\ 1 & x^1_{f1}(t_N+\tau) & x^1_{f2}(t_N+\tau) & ... & x^2_{f1}(t_N+\tau) & x^2_{f2}(t_N+\tau)... \end{pmatrix}$$

On définit aussi le vecteur d'observation **y**(t), qui vaut 1 pendant l'administration d'un stimulus déclenchant ladite première tâche, et 0 autrement : $\mathbf{y} = (y(t_1)\, y(t_2) ... y(t_N))^T$.

On rappelle que $x^i_{f_k}(t_j+\tau)$ représente la composante spectrale dans la bande de fréquence $f_k$ du gradiomètre i mesurée à au - temps $\tau$ suivant l'instant $t_j$ d'enregistrement de la variable d'observation y(t).

Pour calculer le coefficient de corrélation R($\tau$) on effectue d'abord une régression linéaire de y par rapport à X, en écrivant :

$$\hat{y}(t) = b_0 + \sum_{i=1}^{M} b^1_i x^{1}_{fi}(t+\tau) + \sum_{i=1}^{M} b^2_i x^2_{fi}(t+\tau)$$

où le vecteur peut être obtenu par la méthode des moindres carrés, auquel cas $\mathbf{b = (X^TX)^{-1}X^Ty}$

Puis on applique la formule :

$$R^2(\tau) = 1 - \frac{\sum (y(t) - \hat{y}(t))^2}{\sum (y(t) - \bar{y})^2}$$

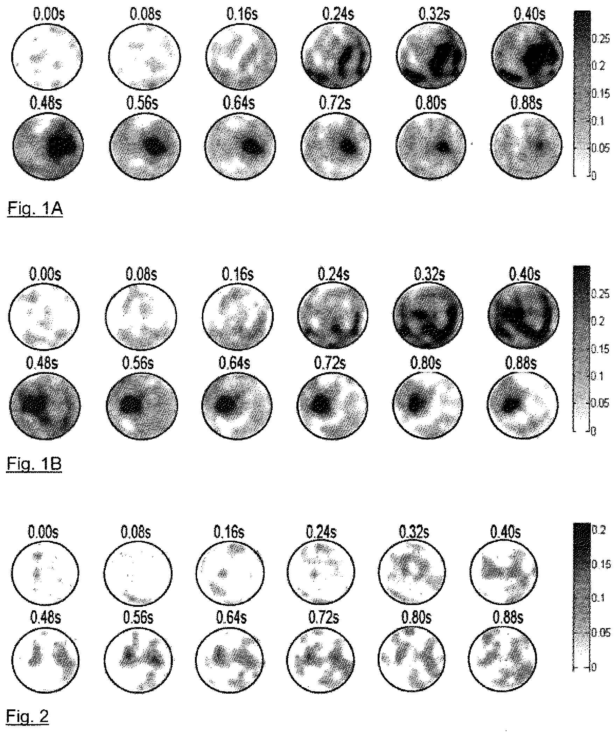

La figure 1A montre des mappes du coefficient de corrélation R($\tau$) ainsi obtenu, pour différentes valeurs du temps $\tau$. La figure 1B montre des mappes obtenues de façon analogue, mais pour une deuxième tâche consistant à imaginer un mouvement de frappe de l'index droit. Sur ces figures, on observe que des corrélations significatives au niveau du cortex postérieur (zone visuelle du cortex - représentée dans la partie supérieure de chaque image), dès $\tau$ = 0,08 s. Cette corrélation correspond à la perception du stimulus visuel par le sujet. Elle est donc sans rapport avec la corrélation que l'on souhaite mettre en évidence, en lien avec la réalisation de la tâche par le sujet. Cette corrélation « utile » est localisée en regard des régions motrices, et non visuelles, du cortex. Ces régions motrices apparaissent sous la forme de zones sombres ponctuelles sur les figures 1A et 1B, pour $\tau$ > 0,48s.

Dans la suite on désignera per $X_L$ et $X_R$ les matrices **X** correspondant aux exemples illustrés sur les figures 1A et 1B, respectivement. Ainsi, $X_L$ correspond à un mouvement imaginaire de l'index gauche, tandis que $X_R$ correspond à un mouvement imaginaire de l'index droit. En outre, on désignera par $Y_L$ et $Y_R$ les vecteurs d'observation y correspondant aux cas illustrés sur les figures 1A et 1B.

Chaque matrice ($X_R$ ou $X_L$) est centrée et normalisée comme suit :

- On identifie les lignes i qui correspondent à y($t_i$) =0, ce qui permet de constituer une sous matrice $X_R$ (respectivement $X_L$), ne comportant que ces lignes i ;
- On détermine la valeur moyenne de chaque colonne de cette sous-matrice, ce qui permet d'avoir une matrice ligne ;
- On soustrait terme à terme cette matrice ligne de chaque ligne de la matrice $X_R$ (respectivement $X_L$);
- On détermine la variance de chaque colonne de la matrice $X'_R$ (respectivement $X'_L$) ainsi obtenue, ce qui permet d'avoir une matrice ligne représentant la variance de chaque colonne ;
- On divise chaque terme d'une colonne de la matrice $X'_R$ (respectivement $X'_L$) par le terme correspondant dans la matrice ligne (normalisation par la variance).

Cela permet de s'affranchir de la « variance physiologique », c'est-à-dire d'une dérive temporelle des signaux mesurés au cours des séries d'acquisitions. Cette étape, optionnelle, n'est pas nécessaire, lorsque les acquisitions sont rapprochées, notamment lorsque les acquisitions sont entrelacées.

On établit ensuite une matrice composite $\mathbf{X}_C$, obtenue en concaténant les matrices $X_R$ et $-X_L$ :

$$Xc = \begin{bmatrix} X_R \\ -X_L \end{bmatrix}$$

ou, plus explicitement :

$$\mathbf{Xc} = \begin{bmatrix} 1 & x_{f1}^{1R}(t_1+\tau) & x_{f2}^{1R}(t_1+\tau) & ... & x_{f1}^{2R}R(t_1+\tau) & x_{f2}^{2R}(t_1+\tau) & ... \\ 1 & x_{f1}^{1R}(t_2+\tau) & x_{f2}^{1R}(t_2+\tau) & ... & x_{f1}^{2R}(t_2+\tau) & x_{f2}^{2R}(t_2+\tau) & ... \\ ... & ... & ... & ... & ... & ... & ... \\ 1 & x_{f1}^{1R}(t_N+\tau) & x_{f2}^{1R}(t_N+\tau) & ... & x_{f2}^{2R}(t_N+\tau) & x_{f2}^{2R}(t_N+\tau) & ... \\ -1 & -x_{f1}^{1L}(t'_1+\tau) & -x_{f2}^{1L}(t'_1+\tau) & ... & -x_{f1}^{2L}(t_1+\tau) & -x_{f2}^{2L}(t_1+\tau) & ... \\ -1 & -x_{f1}^{1L}(t_2+\tau) & -x_{f2}^{1L}(t_2+\tau) & ... & -x_{f1}^{2L}(t_2+\tau) & -x_{f2}^{2L}(t_2+\tau) & ... \\ ... & ... & ... & ... & ... & ... & ... \\ -1 & -x_{f1}^{1L}(t'_M+\tau) & -x_{f2}^{1L}(t_N+\tau) & ... & -x_{f1}^{2L}(t_N+\tau) & -x_{f2}^{2L}(t_N+\tau) ... \end{bmatrix}$$

De même, on établit un vecteur d'observation composite $\mathbf{y}_c$, qui est la concaténation des vecteurs $\mathbf{y}_L$ et $\mathbf{y}_R$,

$$yc = \begin{bmatrix} \mathbf{y}_R \\ \mathbf{y}_L \end{bmatrix}$$

Puis on détermine un coefficient de corrélation $R_c(\tau)$ de $\mathbf{X}_C$ avec $\mathbf{y}_c$ comme précédemment indiqué, La figure 2 montre des mappes de ce coefficient de corrélation « composite » $R_c(\tau)$ pour différentes valeurs de $\tau$. On observe une résolution spatiale améliorée par rapport aux cas des figures 1A et 1B, notamment entre $\tau$ = 0,4 et 0,72 s. Surtout, les corrélations au niveau des régions visuelles du cortex ont disparu.

Les vecteurs $y_R$ et $y_L$ peuvent être indépendants l'un de l'autre, mais généralement de la même taille, ou de tailles comparables.

Par le procédé décrit ci-dessus on aboutit à un coefficient de corrélation par point de mesure (capteur) en fonction du temps $\tau$ entre le stimulus et la mesure. On dispose alors de valeurs de coefficients de corrélation selon un maillage spatial défini par le positionnement des capteurs Il est possible de se baser sur ce maillage pour réaliser une projection desdites valeurs à la surface du cortex. Pour cela, la surface du cortex est obtenue, par exemple à partir de mesures IRM, puis est modélisée. Le maillage formé par les différents capteurs est ensuite recalé par rapport à ce modèle, par exemple en utilisant des repères stéréotaxiques visibles en IRM, notamment des pastilles en sel de gadolinium disposées sur la tête du patient.

A partir des valeurs de coefficients de détermination, on réalise une projection sur le modèle de la surface corticale, la valeur attribuée à chaque élément de ladite surface corticale étant issue d'une interpolation entre différents points du maillage, par exemple les trois plus proches voisins, le critère de pondération étant une distance.

Dans certaines applications on se limite à considérer les valeurs absolues des coefficients de corrélation, leurs signes ne présentant pas d'intérêt. Ainsi, de préférence, des capteurs destinés à réaliser une commande neuronale directe seront placées de préférence en correspondance des régions du cortex présentant les coefficients de corrélation les plus élevés (en valeur absolue) avec les tâches utilisées pour la commande. Il convient de noter que ces capteurs peuvent être différents de ceux utilisés pour la localisation de l'activité cérébrale. Par exemple, des capteurs magnétoencéphalographiques peuvent être utilisés pour localiser l'activité cérébrale (corticale) conformément à l'invention et des électrodes d'ECoG peuvent être utilisées pour la commande neuronale directe.

Plusieurs modes de réalisation peuvent être envisagées. Par exemple :

- Toujours dans le cadre d'un mode de réalisation exploitant la magnétoencéphalographie, les capteurs peuvent être de type différent, et notamment comprendre un magnétomètre en remplacement, ou en complément, des gradiomètres ; de même, une composante du champ magnétique perpendiculaire à la surface du crâne peut également être mesurée.
- D'autres techniques de détection et mesure de l'activité cérébrale peuvent être utilisées, telles que l'électrocorticographie ou l'électroencéphalographie.
- Les deux tâches considérées peuvent ne pas correspondre à des mouvements symétriques du corps du sujet. Il peut également s'agir de mouvements de membres différents (par exemple, mouvement d'un bras et d'une jambe), situés du même côté ou de côtés opposés du corps, ou même de tâches de nature différente, ne correspondant pas (ou dont l'une ne correspond pas) à un mouvement réel ou imaginaire ; par exemple, une tâche peut consister à imaginer une couleur.
- Le centrage et la normalisation des séries de signaux sont avantageux, mais pas essentiels. En outre, des traitements différents de ceux décrits peuvent être appliqués aux signaux afin de déterminer les coefficients de corrélation.
- Le procédé peut être utilisé avec un seul capteur, si l'on souhaite uniquement étudier le degré d'activation d'une région spécifique du cerveau lors de l'effectuation d'une tâche.
- L'invention admet aussi d'autres applications que la commande neuronale directe, par exemple la recherche fondamentale en neurosciences.

## Revendications

1. Procédé de localisation de capteurs d'activité cérébrale pour commande neuronale directe, comportant :

   - une étape de localisation d'une activité cérébrale comportant les étapes suivantes :

      a) Adresser à un sujet une première série de stimuli sensoriels et acquérir, au moyen d'un ensemble de capteurs, des premières séries respectives de signaux représentatifs d'une activité cérébrale associée à une première tâche effectuée ou imaginée par ledit sujet en réponse aux stimuli sensoriels de ladite première série, chaque dit capteur étant sensible à l'activité d'une région respective du cerveau dudit sujet ;
      b) Adresser audit sujet une deuxième série de stimuli sensoriels et acquérir, au moyen dudit ensemble de capteurs, des deuxièmes séries respectives de signaux représentatifs d'une activité cérébrale associée à une deuxième tâche, différente de ladite première tâche, effectuée ou imaginée par ledit sujet en réponse aux stimuli sensoriels de ladite deuxième série ; **caractérisé en ce qu'**il comprend
      l'étape c) consistant **en ce que** pour chaque dit capteur, construire une variable multidimensionnelle représentative de la première et de la deuxième série correspondantes de signaux, et déterminer un coefficient de corrélation entre ladite variable multidimensionnelle et un vecteur d'observation représentatif desdits premier et deuxième stimuli sensoriels,
      ladite étape c) comprend la concaténation des variables multidimensionnelles représentatives desdites première et deuxième série de signaux, avec changement de signe de l'une d'entre elles

   - une étape de détermination de localisations optimales desdits capteurs d'activité cérébrale en fonction des résultats de ladite étape de localisation d'une activité cérébrale.

2. Procédé selon la revendication 1, dans lequel ladite première tâche correspond à un mouvement d'un membre droit du corps dudit sujet et ladite deuxième tâche correspond à un mouvement d'un membre gauche, ou vice-versa.

3. Procédé selon la revendication 2 dans lequel ladite première tâche correspond à un mouvement d'un membre droit du corps dudit sujet et ladite deuxième tâche correspond à un mouvement symétrique du membre gauche correspondant, ou vice-versa.

4. Procédé selon l'une des revendications précédentes, dans lequel ladite étape c) comprend également la réalisation d'une analyse temps-fréquence desdites séries de signaux, moyennant quoi ladite variable multidimensionnelle est une matrice.

5. Procédé selon l'une des revendications précédentes, dans lequel ladite étape c) comprend également une opération de normalisation et centrage desdites séries de signaux.

6. Procédé selon l'une des revendications précédentes, dans lequel lesdits capteurs sont des capteurs magnétoencéphalographiques.

7. Procédé selon la revendication 6 dans lequel chaque dit capteur comprend une paire de gradiomètres agencés pour acquérir deux composantes spatiales distinctes d'un gradient d'un champ magnétique généré par le cerveau dudit sujet.

8. Procédé selon l'une des revendications précédentes, comprenant également une étape d) de visualisation, pendant laquelle on projette des valeurs indicatives des coefficients de corrélations déterminés pour chaque dit capteur sur un modèle tridimensionnel d'une surface corticale, et l'on réalise une interpolation desdites valeurs entre différents points d'un maillage de ladite surface.

**Patentansprüche**

1. Verfahren zum Lokalisieren von Gehirnaktivitätssensoren zur direkten neuronalen Steuerung, umfassend:

   - einen Schritt des Lokalisierens einer Gehirnaktivität, der die folgenden Schritte umfasst:

      a) Adressieren eines Subjekts mit einer ersten Reihe von sensorischen Reizen und Erfassen, mithilfe eines Satzes von Sensoren, einer jeweiligen ersten Reihe von Signalen, die eine zerebrale Aktivität repräsentieren, die mit einer ersten Aufgabe verbunden ist, die von dem Subjekt als Reaktion auf sensorische Reize der ersten Reihe ausgeführt oder vorgestellt wird, wobei jeder Sensor empfindlich ist für die Aktivität einer jeweiligen Region des Gehirns des Subjekts;
      b) Adressieren des Subjekts mit einer zweiten Reihe von sensorischen Reizen und Erfassen, mithilfe des Satzes von Sensoren, der jeweiligen zweiten Reihe von Signalen, die eine zerebrale Aktivität repräsentieren, die mit einer zweiten Aufgabe verbunden ist, die sich von der ersten Aufgabe unterscheidet, die von dem Subjekt als Reaktion auf sensorische Reize der zweiten Reihe ausgeführt oder vorgestellt wird; **dadurch gekennzeichnet, dass** es enthält
      Schritt c), der darin besteht, dass für jeden Sensor eine mehrdimensionale Variable konstruiert wird, die für die entsprechende erste und zweite Reihe von Signalen repräsentativ ist, und ein Korrelationskoeffizient bestimmt wird zwischen der mehrdimensionalen Variable und einem Beobachtungsvektor, derden ersten und zweiten sensorischen Reiz repräsentiert,
      wobei Schritt c) einen Schritt des Verkettens der mehrdimensionalen Variablen umfasst, die die entsprechende erste und zweite Reihe von Signalen repräsentieren, mit Zeichenwechsel bei einem von beiden

   - einen Schritt des Bestimmens der optimalen Positionen der Gehirnaktivitätssensoren als Funktion der Ergebnisse des Schritts des Lokalisierens der Gehirnaktivität.

2. Verfahren nach Anspruch 1, wobei die erste Aufgabe einer Bewegung eines rechten Glieds des Körpers des Subjekts entspricht und die zweite Aufgabe einer Bewegung eines linken Glieds entspricht, oder umgekehrt.

3. Verfahren nach Anspruch 2, wobei die erste Aufgabe einer Bewegung eines rechten Glieds des Körpers des Subjekts entspricht und die zweite Aufgabe einer symmetrischen Bewegung des entsprechenden linken Glieds entspricht, oder umgekehrt.

4. Verfahren nach einem der vorhergehenden Ansprüche, wobei der Schritt c) auch das Durchführen einer Zeit-Frequenz-Analyse der Reihe von Signalen umfasst, wobei die mehrdimensionale Variable eine Matrix ist.

5. Verfahren nach einem der vorhergehenden Ansprüche, wobei der Schritt c) auch einen Vorgang des Normalisierens und Zentrierens der Reihe von Signalen umfasst.

**6.** Verfahren nach einem der vorhergehenden Ansprüche, wobei die Sensoren magnetoenzephalographische Sensoren sind.

**7.** Verfahren nach Anspruch 6, wobei jeder Sensor ein Paar von Gradiometern umfasst, die angeordnet sind, um zwei unterschiedliche räumliche Komponenten eines Gradienten eines Magnetfelds zu erfassen, das vom Gehirn des Subjekts erzeugt wird.

**8.** Verfahren nach einem der vorhergehenden Ansprüche, das auch einen Schritt d) des Visualisierens umfasst, während welchem Werte, die für jeden Sensor bestimmte Korrelationskoeffizienten anzeigen, auf ein dreidimensionales Modell einer kortikalen Oberfläche projiziert werden, und eine Interpolation dieser Werte zwischen verschiedenen Punkten eines Netzes auf der Oberfläche realisiert wird.

**Claims**

**1.** Method for locating brain activity sensors for direct neural control, comprising:

- a step of locating brain activity comprising the following steps:

a) applying to a subject a first series of sensory stimuli and acquiring, by means of a set of sensors, first respective series of signals representative of a brain activity associated with a first task performed or imagined by said subject in response to the sensory stimuli of said first series, each said sensor being sensitive to the activity of a respective region of the brain of said subject;
b) applying to said subject a second series of sensory stimuli and acquiring, by means of said set of sensors, second respective series of signals representative of a brain activity associated with a second task, different from said first task, performed or imagined by said subject in response to the sensory stimuli of said second series; **characterised in that** it comprises:

step c) consisting of, for each said sensor, constructing a multidimensional variable representative of the first and the second corresponding series of signals, and determining a correlation co-efficient between said multidimensional variable and an observation vector representative of said first and second sensory stimuli,
said step c) comprising the concatenation of the multidimensional variables representative of said first and second series of signals, with change of sign of one of them

- a step of determining optimum locations of said brain activity sensors according to the results of said step of locating a brain activity.

**2.** Method according to claim 1, in which said first task corresponds to a movement of a right limb of the body of said subject and said second task corresponds to a movement of a left limb, or vice versa.

**3.** Method according to claim 2, in which said first task corresponds to a movement of a right limb of the body of said subject and said second task corresponds to a symmetrical movement of the corresponding left limb, or vice versa.

**4.** Method according to one of the preceding claims, in which said step c) also comprises the production of a time-frequency analysis of said series of signals, in return for which said multidimensional variable is a matrix.

**5.** Method according to one of the preceding claims, in which said step c) also comprises an operation of standardisation and centring of said series of signals.

**6.** Method according to one of the preceding claims, in which said sensors are magnetoencephalographic sensors.

**7.** Method according to claim 6, in which each said sensor comprises a pair of gradiometers arranged to acquire two distinct spatial components of a gradient of a magnetic field generated by the brain of said subject.

**8.** Method according to one of the preceding claims, also comprising a display step d), during which values indicative of the correlation coefficients determined for each said sensor are projected onto a three-dimensional model of a cortical surface, and an interpolation of said values is produced between different points of a meshing of said surface.

Fig. 1A

Fig. 1B

Fig. 2

**EP 2 950 713 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2992543 A1
- US 5447166 A

- US 20020042563 A1

**Littérature non-brevet citée dans la description**

- **A. BARACHANT ; T. AKSENOVA ; S. BONNET.** Filtrage spatial robuste à partir d'un sous-ensemble optimal d'électrodes en BCI EEG. *GRETSI 2009,* 08 Septembre 2009